# EUROPÄISCHE PATENTSCHRIFT

(11) **EP 0 933 353 B1**
(45) Veröffentlichungstag und Bekanntmachung des Hinweises auf die Patenterteilung: **12.06.2002**
(21) Anmeldenummer: 99101853.2
(22) Anmeldetag: 28.01.1999
(51) Int. Cl.: C07C 67/58, C07C 67/62, C07C 69/54

(54) **Verwendung von anionischen Flockungsmitteln bei der organisch-wässrigen Phasentrennung**
Use of anionic flocculating agents in the organic-aqueous phase separation
Emploi d'agents floculants anioniques dans la séparation de phases organiques et aqueuses

(30) Priorität: 30.01.1998 DE 19803658
(43) Veröffentlichungstag der Anmeldung: 04.08.1999
(73) Patentinhaber: BASF AKTIENGESELLSCHAFT, 67056 Ludwigshafen (DE)
(72) Erfinder: Paulus, Wolfgang, 55128 Mainz (DE); Geisendörfer, Matthias, 67435 Neustadt (DE)
(74) Vertreter: Kinzebach, Werner, Dr.

(56) Entgegenhaltungen:
- EP-A- 0 561 264
- DE-A- 1 954 548
- PATENT ABSTRACTS OF JAPAN vol. 098, no. 004, 31. März 1998 & JP 09 316033 A (TOAGOSEI CO LTD), 9. Dezember 1997
- PATENT ABSTRACTS OF JAPAN vol. 011, no. 229 (C-436), 25. Juli 1987 & JP 62 042948 A (ASAHI CHEM IND CO LTD), 24. Februar 1987

## Beschreibung

Die Herstellung von (Poly)esteracrylaten, (Poly)etheracrylaten oder monomeren Acrylaten erfolgt üblicherweise durch Veresterung von (Poly)esterpolyolen, (Poly)etherpolyolen oder Alkanolen mit α,β-ethylenisch ungesättigten Carbonsäuren oder deren Anhydriden. Entsprechende Herstellungsverfahren sind beispielsweise in N. S. Allen, M. A. Johnson, P. Oldring (Hrsg.) und M. S. Salim, Chemistry & Technology of UV & EB-Curing Formulations for Coatings, Inks & Paints, Vol. 2, SITA Technology, London 1991, beschrieben.

Die Veresterung ist eine Gleichgewichtsreaktion, bei der das Gleichgewicht üblicherweise unter anderem dadurch in Richtung der Esterbildung verschoben wird, dass man die Carbonsäure im Überschuss zugibt. üblicherweise entfernt man nach der Veresterung überschüssige α,β-ethylenisch ungesättigte Carbonsäure, die beispielsweise wegen ihres Geruchs oder ihrer Reizwirkung die Verwendbarkeit des Veresterungsproduktes beeinträchtigen könnte, indem man die überschüssige Säure destillativ entfernt oder das Reaktionsgemisch ein- oder mehrmals mit wässrigen Medien wäscht.

Das Waschverfahren hat jedoch den gravierenden Nachteil, dass sich die das Veresterungsprodukt enthaltende organische Phase nur langsam von der die überschüssige Carbonsäure enthaltenden wässrigen Phase trennt. Die Vervollständigung der Phasentrennung erfordert lange Standzeiten, die bei der Herstellung von monomeren Acrylaten, (Poly)esteracrylaten oder (Poly)etheracrylaten im großtechnischen Maßstab mehrere Stunden betragen können. Die Herstellung dieser Acrylate wird dadurch erheblich verteuert.

Üblicherweise setzt man zur Beschleunigung der Phasentrennung zwischen organischen und wässrigen Phasen sogenannte Emulsionsbrecher oder Demulgatoren ein. Beispiele für solche Emulsionsbrecher sind niedermolekulare Verbindungen, wie Salze von Fettsäuren oder Sulfonsäuren sowie die Säuren selbst und höhermolekulare oberflächenaktive Produkte auf Basis von Ethylenoxid- oder Propylenoxidaddukten (vgl. Römpps Chemie-Lexikon, 7.Aufs., Stuttgart 1975, S.778 und 3180). Letztere sind nichtionogene Verbindungen, wie z. B. die unter der Handelsbezeichnung Separol® von der BASF AG, Ludwigshafen vertriebenen Substanzen. Von der Anmelderin durchgeführte Versuche mit solchen Emulsionsbrechern zeigten jedoch, dass sie keine Beschleunigung der Phasentrennung zwischen organischen Phasen, die die oben genannten Acrylate enthalten, und wässrigen Phasen, welche die zur Acrylatherstellung eingesetzten Alkohole, Carbonsäuren bzw. Anhydride enthalten, bewirken.

Die Möglichkeit einer Verwendung von Flockungsmitteln zur Beeinflussung der Phasentrennung wurde im Stand der Technik bisher nicht diskutiert. Flockungsmittel werden üblicherweise zu völlig anderen Zwecken eingesetzt, nämlich um das sogenannte Zeta-Potential kolloidal gelöster Teilchen so zu beeinflussen, dass ein Ausflocken der Feststoffteilchen aus der kolloidalen Flüssigkeit bewirkt wird.

Der vorliegenden Erfindung liegt die Aufgabe zugrunde, die Phasentrennzeiten organisch-wässriger Lösungssysteme bei der Herstellung von monomeren Acrylaten, (Poly)esteracrylaten oder (Poly)etheracrylaten gegenüber dem Stand der Technik zu verkürzen.

Überraschenderweise wurde gefunden, dass die Zugabe eines organischen, polymeren, anionischen wasserlöslichen Flockungsmittels zur Verkürzung der Phasentrennzeiten zwischen monomeren Acrylaten, (Poly)esteracrylate oder (Poly)etheracrylate enthaltenden organischen Phasen und wässrigen Phasen, insbesondere dem zum Entfernen überschüssiger Reaktanden der Acrylatherstellung verwendeten wässrigen Waschmedium, führt.

Gegenstand der vorliegenden Erfindung ist daher die Verwendung von organischen, polymeren, anionischen wasserlöslichen Flokkungsmitteln bei der Phasentrennung eines organisch-wässrigen Systems, das im Wesentlichen monomere Acrylate, Diacrylate, organische (Poly)esteracrylate oder (Poly)etheracrylate und nichtumgesetzte Reste der für deren Herstellung verwendeten Reaktanden, insbesondere Alkohole und Carbonsäuren oder Carbonsäureanhydride, enthält.

Die oben genannten organischen Acrylat-Komponenten und nichtumgesetzten Anteile von Alkoholen und Carbonsäuren oder Anhydriden liegen üblicherweise gelöst in dem organisch-wässrigen System vor. Im Gegensatz zur Acrylat-Komponente besitzen die ebenfalls enthaltenen Alkohole bzw. Carbonsäuren und Anhydride jedoch eine deutlich höhere Löslichkeit in wässrigen Medien.

Üblicherweise spricht der Fachmann auch dann von Ester-, Ether- oder monomeren Acrylaten, wenn deren Ausgangsverbindungen nicht mit Acrylsäure, sondern mit einer anderen α,β-ethylenisch ungesättigten Carbonsäure verestert wurden. Im Rahmen der vorliegenden Erfindung wird diese Terminologie beibehalten.

Die erfindungsgemäße Verwendung weist gegenüber dem Stand der Technik einige überraschende Vorteile auf:
I. Das Entfernen überschüssiger α,β-ethylenisch ungesättigter Carbonsäure kann durch die Verkürzung der Phasentrennzeiten erheblich beschleunigt werden.
II. Der Einsatz anionischer Flockungsmittel erhöht die erzielbare Ausbeute, da die Flockungsmittel die Ausbildung einer klaren Phasengrenze bewirken.
III. Die anwendungstechnischen Eigenschaften der Oligo-/Polyesteracrylate oder Oligo-/Polyetheracrylate, wie Formulierbarkeit und mechanische Eigenschaften der UV-gehärteten Filme werden durch den Zusatz anionischer Flockungsmittel nicht beeinträchtigt.

### Brauchbare anionische Polymere:

Erfindungsgemäß verwendet man vorzugsweise wasserlösliche anionische Flockungsmittel, die ausgewählt sind unter funktionalen hochmolekularen Polymeren, die anionischen Carbonsäure-, Sulfonsäure-, Phosphorsäure- und/oder Phosphonsäurefunktionen, insbesondere Carbonsäurereste, in neutralisierter Form oder in Salzform sowie gegebenenfalls weitere polare Reste, wie Carbonsäureamidreste enthalten. Erfindungsgemäß geeignete anionisch modifizierte Flockungsmittel sind mit Hilfe herkömmlicher Polymerisationsverfahren dadurch erhältlich, dass man wenigstens ein ethylenisch ungesättigtes, eine anionische oder anionisierbare Seitengruppe tragendes Monomer, beispielsweise ausgewählt unter Acrylsäure, Methacrylsäure, Vinylsulfonsäure, Vinylphosphonsäure, Itaconsäure, 2-Acrylamidomethylpropansulfonsäure, Sulfopropylacrylat und Sulfopropylmethacrylat, mit wenigstens einem weiteren nicht-ionischen Comonomeren, wie z. B. ausgewählt unter Acrylamid, Methacrylamid, N-Vinylformamid, N-Vinylacetamid, N-Vinylmethylacetamid, N-Vinylmethylformamid, Vinylacetat und Vinylpyrrolidon, copolymerisiert. Weiterhin wäre es denkbar, anionische funktionale Gruppen durch Veresterung von Carboxylgruppen mit einem Polyol, z. B. Ethandiol, und weitere Umsetzung der verbleibenden freien Hydroxylgruppen, z. B. mit Schwefelsäure oder Phosphorsäure, in das hochmolekulare Polymer einzuführen. Insbesondere verwendet man erfindungsgemäß anionisch modifiziertes, hochmolekulares Polyacrylamid, beispielsweise erhältlich durch Copolymerisation von (Meth)Acrylamid und (Meth)acrylsäure.

Im Rahmen der vorliegenden Erfindung bevorzugt verwendbare anionische Flockungsmittel weisen bei einer Konzentration von 0,01 % Polymer in VE-Wasser eine Viskosität im Bereich von 11 bis 13, insbesondere 12 bis 12,5 mPa·s, gemessen mit einem Rheomat 30 der Fa. Contraves, auf. Vorzugsweise verwendet man anionische Flokkungsmittel, die eine Ladungsdichte im Bereich von 1 bis 10, insbesondere von 4 bis 9 (Milliäquivalent Na-Acrylat pro Gramm Substanz), aufweisen. Das Molekulargewicht der bevorzugt verwendbaren anionischen Flockungsmittel liegt im Bereich von 4 bis 22, insbesondere von 10 bis 20 Megadalton. Besonders geeignete Flokkungsmittel sind die unter der Handelsbezeichnung Sedipur® von der BASF AG, Ludwigshafen vertriebenen, anionischen Produkte.

Vorzugsweise verwendet man die anionischen Flockungsmittel in einer Konzentration von etwa 10 bis 1000 ppm, besonders bevorzugt etwa 50 bis 200 ppm, bezogen auf das wässrige Waschmedium.

Die wässrige Komponente (Phase) des organisch-wässrigen Systems enthält vorzugsweise ionische Verbindungen, wie Alkali- oder Erdalkalimetallhalogenide, insbesondere NaCl, CaCl₂, MgSO₄ oder KCl in Mengen von etwa 0,1 bis 30, insbesondere etwa 1 bis 25 Gew.-%, bezogen auf das Gesamtgewicht der wässrigen Komponente, um die Phasentrennung zu beschleunigen. Es ist außerdem bevorzugt, der wässrigen Komponente basische Verbindungen, beispielsweise NaOH, Ca(OH)₂ oder KOH, zuzusetzen, um die überschüssige α,β-ethylenisch ungesättigte Carbonsäure zu neutralisieren. Die wässrige Komponente stellt eine Waschphase für die organische, acrylathaltige Phase dar. Die Waschphase kann der organischen Phase ein- oder mehrmals mit gleicher oder wechselnder Zusammensetzung zugegeben werden.

Der erfindungsgemäße Einsatz der anionischen Polymere bietet sich vor allem dort an, wo in der organischen Phase gut lösliche Acrylate im Gemisch mit besser wasserlöslichen Verbindungen, wie Carbonsäure, anfallen. Als Beispiele hierfür können genannt werden:
a) Die Herstellung von Polyester- und Polyetheracrylaten durch Kondensation von α,β-ethylenisch ungesättigten Carbonsäuren mit Alkanolen, wie Polyesterolen bzw. Polyetherolen; wie z. B. von Polymeren, die unter der Handelsbezeichnung Laromer® von der BASF AG, Ludwigshafen vertrieben werden.
b) Die Herstellung niedermolekularer Acrylatverbindungen in Waschfahrweise.

Je nach Einsatzgebiet sind demzufolge unterschiedliche Stoffgemische zu trennen.

Gemäß einem ersten Anwendungsgebiet der Erfindung umfasst die organische Komponente (Phase) des organisch-wässrigen Systems vorzugsweise Polyesteracrylate oder Polyetheracrylate, die durch Kondensation von Alkanolen, insbesondere Polyesterolen bzw. Polyetherolen, mit α,β-ethylenisch ungesättigten Carbonsäuren oder deren Anhydriden oder von endständige Carboxylgruppen tragenden Polyestern mit Hydroxylgruppen aufweisenden Estern der Carbonsäuren in organischer Phase erhältlich sind. Für die Herstellung der Polyester- bzw. Polyetheracrylate brauchbare Reaktanden werden in den folgenden Abschnitten näher erläutert.

### α,β-ethylenisch ungesättigte Carbonsäuren:

Als α,β-ethylenisch ungesättigte Carbonsäuren und deren Anhydride können Mono- und/oder Dicarbonsäuren, z. B. Acrylsäure, Methacrylsäure, Fumarsäure, Maleinsäure, Maleinsäureanhydrid, Crotonsäure, Itaconsäure, etc. oder Gemische davon eingesetzt werden. Bevorzugt werden Acrylsäure und Methacrylsäure eingesetzt.

### Polyesterole:

Geeignete einsetzbare Polyesterole sind lineare und verzweigte Polymere mit endständigen OH-Gruppen, z. B. solche mit zwei OH-Endgruppen.

Die Polyesterole lassen sich in einfacher Weise durch Veresterung von aliphatischen, cycloaliphatischen und aromatischen Di-, Tri- und/oder Polycarbonsäuren mit Di-, Tri- und/oder Polyolen herstellen.

Zur Herstellung der Polyesterole geeignete Carbonsäuren sind z. B. Dicarbonsäuren mit 2 bis 20 Kohlenstoffatomen, vorzugsweise 4 bis 15 Kohlenstoffatomen, beispielsweise Malonsäure, Bernsteinsäure, Adipinsäure, Glutarsäure, Pimelinsäure, Korksäure, Sebacinsäure, Dodecandisäure, Phthalsäure, Terephthalsäure, Isophthalsäure, Cyclohexandicarbonsäure, etc. Die Dicarbonsäuren können einzeln oder als Gemische eingesetzt werden.

Zur Herstellung der Polyesterole geeignete Diole sind z. B. Glykole, bevorzugt Glykole mit 2 bis 25 Kohlenstoffatomen. Beispiele für geeignete Glykole sind z. B. 1,2-Ethandiol, 1,3-Propandiol, 1,4-Butandiol, 1,5-Pentandiol, 1,6-Hexandiol, 1,10-Decandiol, Diethylenglykol, Dipropylenglykol, Tripropylenglykol, 2,2,4-Trimethylpentandiol-1,5, 2,2-Dimethylpropandiol-1,3, 1,4-Dimethylolcyclohexan, 1,6-Dimethylolcyclohexan und ethoxylierte/propoxylierte Ether und ethoxylierte/propoxylierte Produkte des 2,2-Bis-(4-hydroxyphenyl)-propan (Bisphenol-A), etc. Geeignete Triole und Polyole weisen z. B. 3 bis 25, bevorzugt 3 bis 18 Kohlenstoffatome auf. Dazu zählen z. B. Glycerin, Trimethylolpropan, Erythrit, Pentaerythrit, Sorbit, etc sowie die ethoxylierten/propoxylierten Produkte davon. Geeignete Polyesterole lassen sich ebenfalls durch Polymerisation von Lactonen, z. B. Lactonen mit 3 bis 20 Kohlenstoffatomen, herstellen. Als Lactone für die Herstellung der Polyesterole eignen sich z. B. α,α-Dimethyl-β-propiolacton, γ-Butyrolacton, ε-Caprolacton, etc.

Bei der Herstellung der Polyesteracrylate sind ferner Kondensationsprodukte auf Basis von Hydroxylgruppen aufweisenden Estern α,β-ethylenisch ungesättigter Mono- und/oder Dicarbonsäuren, insbesondere der Acrylsäure und/oder Methacrylsäure, mit mindestens zweiwertigen Alkoholen einsetzbar. Dazu zählen z. B. 2-Hydroxyethylacrylat, 2-Hydroxy-ethylmethacrylat, 2-Hydroxypropylacrylat, 2-Hydroxypropylmethacrylat, 3-Hydroxybutylacrylat, 3-Hydroxybutylmethacrylat, 4-Hydroxybutylacrylat, 4-Hydroxybutylmethacrylat, 6-Hydroxyhexylacrylat, 6-Hydroxyhexylmethacrylat, 3-Hydroxy-2-ethylhexylacrylat, 3-Hydroxy-2-ethylhexylmeth-acrylat, Di(meth)acrylsäureester des 1,1,1-Trimethylolpropans oder des Glycerins. Diese Hydroxylgruppen aufweisenden Ester können mit endständige Carboxylgruppen tragenden Polyestern kondensiert werden. Die endständige Carboxylgruppen tragenden Polyester sind in üblicher Weise z. B. durch Veresterung der oben genannten Di-, Tri- und/oder Polyole mit Di-, Tri- und/oder Polycarbonsäuren im Überschuss erhältlich.

### Polyetherole:

Als Polyetherole können lineare oder verzweigte endständige Hydroxylgruppen aufweisende Substanzen eingesetzt werden, die Etherbindungen enthalten und ein Molekulargewicht im Bereich von z. B. etwa 250 bis 10 000, vorzugsweise von 250 bis 5000 Dalton, besitzen.

Geeignete Polyetherole können leicht durch Polymerisation von cyclischen Ethern, wie Tetrahydrofuran, oder durch Umsetzung von einem oder mehreren Alkylenoxiden mit 2 bis 4 Kohlenstoffatomen im Alkylrest mit einem Startermolekül, das zwei aktive Wasserstoffatome enthält, hergestellt werden. Als Alkylenoxide seien beispielsweise Ethylenoxid, 1,2-Propylenoxid, Epichlorhydrin, 1,2- und 2,3-Butylenoxid genannt. Die Alkylenoxide können einzeln, alternierend nacheinander oder als Mischung eingesetzt werden. Als Startermolekül kommen z. B. Wasser, die zuvor genannten Glykole oder Triole, Amine, wie Ethylendiamin, Hexamethylendiamin und 4,4'-Diaminodiphenylmethan sowie Aminoalkohole, wie Ethanolamin, in Betracht.

Ebenso wie die Polyesterole können auch die Polyetherole allein oder in Mischungen verwendet werden.

Die Erfindung ist insbesondere brauchbar zur Aufarbeitung eines Reaktionsgemisches das alkoxyliertes, insbesondere ethoxyliertes und/oder propoxyliertes Trimethylolpropantriacrylat mit einem Alkoxylierungsgrad im Bereich von 3 bis 30, enthält.

Gemäß einem zweiten Anwendungsgebiet der Erfindung umfasst die organische Komponente (Phase) niedermolekulare Acrylatester, welche im Waschfahrweise aus (Meth)Acrylsäure und (Poly)Alkoholen herstellbar sind. Als Beispiele sind zu nennen monomere Acrylate, wie beispielsweise tert.-Butyl-cyclohexylacrylat, Laurylacrylat, 2-Ethylhexylacrylat, Decandioldiacrylat, Hexandioldiacrylat, Butandioldiacrylat, Tripropylenglykoldiacrylat, Dipropylenglykoldiacrylat, Ethyldiglykolacrylat, Neopentylglykoldiacrylat, Trimethylolpropantriacrylat, Pentaerythrittriacrylat sowie Pentaerythrittetraacrylat.

Bei den oben beschriebenen Veresterungsreaktionen zur Herstellung der in der organischen Komponente des organisch-wässrigen Systems enthaltenen Acrylate sind als Lösungsmittel insbesondere solche organische Lösungsmittel einsetzbar, deren Siedepunkt unter 110 °C liegt und die mit Wasser azeotrope Gemische bilden. Besonders geeignete organische Lösungsmittel sind Pentan, Hexan, Heptan, Cyclohexan, Methylcyclohexan, Cyclopentan, Dichlorethan, Toluol, Xylol sowie Ligroin und Petrolether.

Vorzugsweise wird die Veresterungsreaktion in Gegenwart starker Säuren als Veresterungskatalysatoren durchgeführt. Geeignete Veresterungskatalysatoren sind beispielsweise Schwefelsäure, Phosphorsäure, Alkylsulfonsäuren, Arylsulfonsäuren oder Kationenaustauscher mit Sulfonsäuregruppen, insbesondere jedoch Schwefelsäure, besonders bevorzugt Toluolsulfonsäuren.

Die Katalysatorkonzentration im Reaktionsgemisch beträgt in der Regel 0,1 bis 10 Gew.-%, vorzugsweise 0,5 bis 5 Gew.-%.

Vorzugsweise wird die Veresterung in Gegenwart von üblichen Polymerisationsinhibitoren oder von Metallsalzen in einer Menge von jeweils etwa 100 bis 5000 ppm, bezogen auf das Reaktionsgemisch, oder gegebenenfalls Magerluft mit einem O₂-Gehalt ≤ 6 % (0,1 bis 1000 l pro Stunde ·l) durchgeführt. Die Reaktionstemperatur für die Veresterung liegt im Allgemeinen bei etwa 70 bis 160 °C, vorzugsweise bei 90 bis 130 °C.

Die Reaktionszeit beträgt für die Veresterungsreaktion im Allgemeinen 1 bis 24, vorzugsweise 1 bis 16, insbesondere 2 bis 12 Stunden.

Die Reaktion kann unter Normal-, Unter- und Überdruck durchgeführt werden. Vorzugsweise wird der Druck so eingestellt, dass während der Veresterung das gebildete Wasser abdestilliert, z. B. in Form eines azeotropen Gemisches aus Wasser und organischem Lösungsmittel, wobei die organische Komponente vorzugsweise wieder der Veresterung zugeführt wird. Die Veresterung kann kontinuierlich oder diskontinuierlich durchgeführt werden, wobei die diskontinuierliche Reaktionsdurchführung bevorzugt ist.

Die Veresterung wird in üblichen Apparaturen durchgeführt, z. B. in einer Veresterungseinheit aus einem oder mehreren beheizbaren Rührreaktoren (Kaskade), die gegebenenfalls mit Kolonnen, Kondensatoren und Phasentrenngefäßen ausgerüstet sind. Der Reaktorinhalt wird durch Rühren, Zwangsumlauf oder andere übliche und geeignete Maßnahmen durchmischt.

Das nach der Veresterung erhaltene Reaktionsgemisch wird durch einen oder mehrere Waschschritte in Gegenwart eines oben beschriebenen anionischen Flockungsmittels von überschüssiger, α,β-ethylenisch ungesättigter Carbonsäure befreit. So kann beispielsweise ein dreistufiger Waschschritt durchgeführt werden, indem man das Reaktionsgemisch der Veresterung zunächst mit einer NaCl-haltigen wässrigen Phase wäscht, anschließend mit einer NaOH-haltigen wässrigen Phase neutralisiert und schließlich erneut mit einem NaCl-haltigen wassrigen Medium nachwäscht.

vorzugsweise wird vor jedem Waschschritt die jeweils einzusetzende Menge an organischem Flockungsmittel dem wässrigen Waschmedium zugesetzt und erst dann das Waschmedium mit dem Flockungsmittel dem nach der Veresterung erhaltenen Reaktionsgemisch zugegeben. Das Flockungsmittel kann jedoch auch dem wässrig-organischen System direkt zugegeben werden. Die gesamte einzusetzende Menge an organischem Flockungsmittel kann auf einmal zugesetzt oder auch langsam zudosiert werden. Das nach jedem Waschschritt erhaltene, wässrig-organische System wird jeweils in Ruhe belassen, bis die Phasentrennung erfolgt ist. Die Phasentrennung wird durch die erfindungsgemäße Verwendung des anionischen Flockungsmittels erheblich beschleunigt.

Der Waschvorgang wird in der Regel in einer üblichen Extraktionsapparatur, beispielsweise in einem Rühr- oder Waschkessel, einer Kolonne ohne Energieeintrag, einer gepulsten Kolonne, einer Kolonne mit rotierendem Einsatz, einer Mixer-Settler-Apparatur oder einem Zentrifugalextraktor, vorzugsweise in einem Waschkessel, durchgeführt. Geeignete Apparaturen sind beispielsweise in Ullmann's Encyclopedia of Industrial Chemistry, 5th ed., CD-ROM-Version, Kap. 3.1.1 bis Kap. 3.1.5 beschrieben.

Ein weiterer Gegenstand der vorliegenden Erfindung ist ein Verfahren zur Herstellung von Acrylaten durch
a) Kondensation von Alkanolen gemäß obiger Definition mit α,β-ethylenisch ungesättigten Carbonsäuren oder deren Anhydriden gemäß obiger Definition in einem organischen Reaktionsmedium und
b) Waschen der organischen Phase mit einem wässrigen Waschmedium, insbesondere zum Entfernen überschüssiger α,β-ethylenisch ungesättigter Carbonsäure,
das dadurch gekennzeichnet ist, dass man Schritt b) unter Verwendung eines anionischen Flockungsmittels gemäß der obigen Definition durchführt.

Schritt a) wird vorzugsweise wie oben für die Herstellung von Polyesteracrylaten und Polyetheracrylaten erläutert durchgeführt.

Die folgenden Beispiele und Vergleichsbeispiele erläutern die Erfindung, ohne sie jedoch darauf einzuschränken:

### Allgemeine Versuchsdurchführung

In einem Waschgefäß wurden 3,7 Liter eines Reaktionsgemisches, das 40 Gew.-% Cyclohexan als Lösungsmittel, etwa 55 Gew.-% alkoxyliertes Trimethylolpropantriacrylat und etwa 5 Gew.-% Acrylsäure enthielt, in drei Waschschritten, nämlich Vorwäsche (mit 1 Liter), Neutralisation (mit 0,67 Liter) und Nachwäsche (mit 1 Liter) wässrigem Waschmedium von überschüssiger Acrylsäure befreit. Das bei der Vorwäsche eingesetzte Waschmedium enthielt Wasser sowie zur Erleichterung der Phasentrennung NaCl. Das zur Neutralisation eingesetzte Waschmedium enthielt Wasser und NaOH und das zur Nachwäsche eingesetzte Waschmedium Wasser und NaCl. Tabelle 1 zeigt vier Beispiele (1 - 4) für die Verringerung der Phasentrennzeiten durch den Einsatz anionischer Flockungsmittel in einer Konzentration von 100 bzw. 80 ppm in allen drei Waschschritten. Tabelle 1 gibt gleichfalls 4 Vergleichsbeispiele (V1 - V4) an, nämlich ein Vergleichsexperiment ohne Flockungsmittel (Referenz), eines mit einem nicht-ionischen Flockungsmittel, eines mit einem kationischen Flockungsmittel und eines mit einem nichtionischen Emulsionsbrecher.

Es ist klar ersichtlich, dass der Einsatz des anionischen Flokkungsmittels die Phasentrennzeiten deutlich verkürzt.

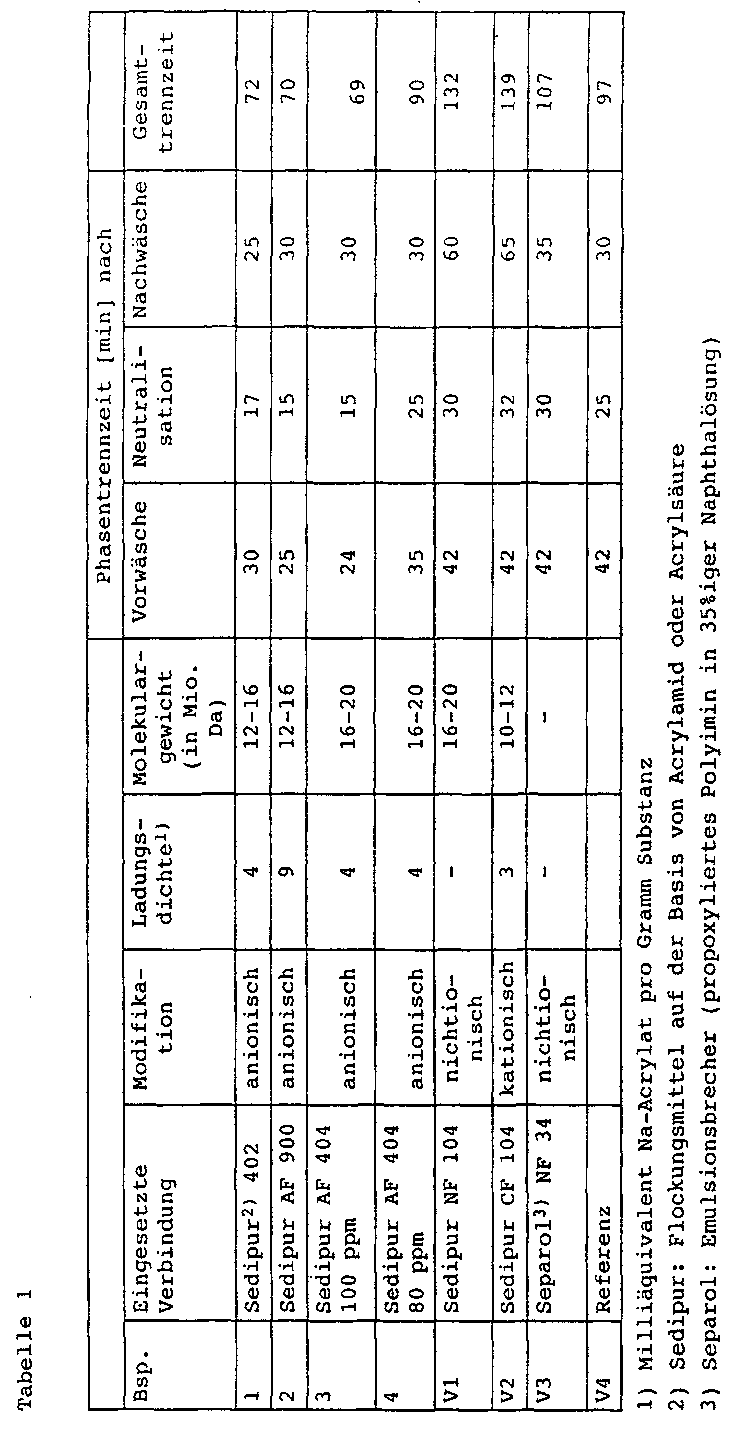

## Patentansprüche

1. Verwendung von organischen, polymeren, anionischen wasserlöslichen Flockungsmitteln bei der Phasentrennung eines organisch-wässrigen Systems, das im Wesentlichen monomere Acrylate, Diacrylate, (Poly)esteracrylate oder (Poly)etheracrylate und nicht-umgesetzte Reste der für die Acrylat-Herstellung verwendeten Alkohole, Carbonsäuren oder Carbonsäureanhydride enthält.

2. Verwendung nach Anspruch 1, **dadurch gekennzeichnet, dass** das Flockungsmittel ausgewählt ist unter funktionalen hochmolekularen Polymeren, die Carbonsäure-, Sulfonsäure-, Phosphorsäure- und/oder Phosphonsäurefunktionen enthalten.

3. Verwendung nach einem der Ansprüche 1 oder 2, **dadurch gekennzeichnet, dass** es sich bei dem Flockungsmittel um ein anionisch modifiziertes Polyacrylamid handelt.

4. Verwendung nach einem der vorhergehenden Ansprüche, **dadurch gekennzeichnet, dass** das anionische Flockungsmittel ein Molekulargewicht von 4 bis 22 Megadalton aufweist.

5. Verwendung nach einem der vorhergehenden Ansprüche, **dadurch gekennzeichnet, dass** das anionische Flockungsmittel bei einer-Konzentration von 0,01 Gew.-% Polymer in vollentsalztem Wasser eine Viskosität im Bereich von 11 bis 13 mPa·s, aufweist.

6. Verwendung nach einem der vorhergehenden Ansprüche, **dadurch gekennzeichnet, dass** das anionische Flockungsmittel eine Ladungsdichte im Bereich von 1 bis 10 Milliäquivalent Natriumacrylat pro g Substanz aufweist.

7. Verwendung nach einem der vorhergehenden Ansprüche, **dadurch gekennzeichnet, dass** das anionische Flockungsmittel in einer Konzentration von 10 bis 1000 ppm, bezogen auf die wässrige Phase des organisch-wässrigen Systems, eingesetzt wird.

8. Verwendung nach einem der vorhergehenden Ansprüche, **dadurch gekennzeichnet, dass** die organische Phase des organisch-wässrigen Systems Polyetheracrylate enthält.

9. Verwendung nach einem der vorhergehenden Ansprüche, **dadurch gekennzeichnet, dass** die organische Phase des organisch-wässrigen Systems alkoxyliertes Trimethylolpropantriacrylat enthält.

10. Verfahren zur Herstellung von Acrylaten durch
a) Kondensation wenigstens eines Alkanols mit wenigstens einer α,β-ethylenisch ungesättigten Carbonsäure oder Anhydrid davon in organischem Medium und
b) Waschen der organischen Phase mit einem wässrigen Waschmedium,
**dadurch gekennzeichnet, dass** man Schritt b) unter Verwendung eines Flockungsmittels gemäß der Definition in einem der Ansprüche 1 bis 6 durchführt.

## Claims

1. The use of organic, polymeric, anionic water-soluble flocculants for the phase separation of an organic-aqueous system which essentially comprises monomeric acrylates, diacrylates, (poly)ester acrylates or (poly)ether acrylates and unreacted residues of the carboxylic anhydrides, carboxylic acids or alcohols used for preparing the acrylates.

2. The use as claimed in claim 1, wherein the flocculant is selected from functional, high molecular mass polymers which comprise carboxylic acid, sulfonic acid, phosphoric acid and/or phosphonic acid functions.

3. The use as claimed in either of claims 1 and 2, wherein the flocculant is an anionically modified polyacrylamide.

4. The use as claimed in any of the preceding claims, wherein the anionic flocculant has a molecular weight of from 4 to 22 megadaltons.

5. The use as claimed in any of the preceding claims, wherein the anionic flocculant has a viscosity in the range from 11 to 13 mPa·s at a concentration of 0.01% by weight of polymer in fully deionized water.

6. The use as claimed in any of the preceding claims, wherein the anionic flocculant has a charge density in the range from 1 to 10 milliequivalents of sodium acrylate per g of substance.

7. The use as claimed in any of the preceding claims, wherein the anionic flocculant is employed in a concentration of from 10 to 1000 ppm based on the aqueous phase of the organic-aqueous system.

8. The use as claimed in any of the preceding claims, wherein the organic phase of the organic-aqueous system comprises polyether acrylates.

9. The use as claimed in any of the preceding claims, wherein the organic phase of the organic-aqueous system comprises alkoxylated trimethylolpropane triacrylate.

10. A process for preparing acrylates by
a) condensing at least one alkanol with at least one α,β-ethylenically unsaturated carboxylic acid or anhydride thereof in organic medium and
b) washing the organic phase with an aqueous washing medium
which comprises conducting step b) using a flocculant as defined in any of claims 1 to 6.

## Revendications

1. Utilisation de floculants organiques, polymères, anioniques, solubles dans l'eau, lors de la séparation des phases d'un système organo-aqueux, qui contient pour l'essentiel des acrylates monomères, des diacrylates monomères, des (poly)esteracrylates ou (poly)étheracrylates, et des résidus n'ayant pas réagi des alcools, acides carboxyliques ou anhydrides carboxyliques utilisés pour la préparation des acrylates.

2. Utilisation selon la revendication 1, **caractérisée en ce que** le floculant est choisi parmi les polymères fonctionnels à grande masse moléculaire qui contiennent des fonctions acide carboxylique, acide sulfonique, acide phosphorique et/ou acide phosphonique.

3. Utilisation selon l'une des revendications 1 ou 2, **caractérisée en ce que**, pour ce qui concerne le floculant, il s'agit d'un polyacrylamide à modification anionique.

4. Utilisation selon l'une des revendications précédentes, **caractérisée en ce que** le floculant anionique a une masse moléculaire de 4 à 22 mégadaltons.

5. Utilisation selon l'une des revendications précédentes, **caractérisé en ce que** le floculant anionique présente, à une concentration de 0,01 % en poids de polymère dans de l'eau entièrement déminéralisée, une viscosité de 11 à 13 mPa.s.

6. Utilisation selon l'une des revendications précédentes, **caractérisée en ce que** le floculant anionique a une densité de charge de 1 à 10 milliéquivalents d'acrylate de sodium par gramme de substance.

7. Utilisation selon l'une des revendications précédentes, **caractérisée en ce que** le floculant anionique est utilisé à une concentration de 10 à 1000 ppm par rapport à la phase aqueuse du système organo-aqueux.

8. Utilisation selon l'une des revendications précédentes, **caractérisée en ce que** la phase organique du système organo-aqueux contient des polyétheracrylates.

9. Utilisation selon l'une des revendications précédentes, **caractérisée en ce que** la phase organique du système organo-aqueux contient du triacrylate de triméthylolpropane alcoxylé.

10. Procédé de préparation d'acrylates, par
a) condensation d'au moins un alcanol avec au moins un acide ou un anhydride carboxylique à insaturation α,β-éthylénique en milieu organique, et
b) lavage de la phase organique avec un milieu de lavage aqueux,
**caractérisé en ce qu'**on met en oeuvre l'étape b) par utilisation d'un floculant tel que défini dans l'une des revendications 1 à 6.
